(19) Europäisches Patentamt
European Patent Office
Office européen des brevets

(11) Veröffentlichungsnummer: **0 071 165**
**B1**

# (12) EUROPÄISCHE PATENTSCHRIFT

(45) Veröffentlichungstag der Patentschrift: **04.09.85**

(51) Int. Cl.⁴: **A 61 K 7/06**, A 61 K 7/48

(21) Anmeldenummer: **82106562.0**

(22) Anmeldetag: **21.07.82**

## (54) Ungesättigte Arylketone als antiseborrhoische Zusätze für kosmetische Mittel.

(30) Priorität: **29.07.81 DE 3129867**

(43) Veröffentlichungstag der Anmeldung:
**09.02.83 Patentblatt 83/6**

(45) Bekanntmachung des Hinweises auf die Patenterteilung:
**04.09.85 Patentblatt 85/36**

(84) Benannte Vertragsstaaten:
**AT BE CH DE FR GB IT LI LU NL SE**

(56) Entgegenhaltungen:
**DD - A - 69 672**
**US - A - 1 732 120**
**US - A - 3 972 996**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Möller, Hinrich, Dr., Schumannstrasse 11, D-4019 Monheim (DE)**
Erfinder: **Wallat, Siegfried, Dr., Hasenstrasse 7, D-4019 Monheim (DE)**
Erfinder: **Bartnik, Friedhelm, Dr., Melanchthonstrasse 11, D-4000 Düsseldorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 165 B1

ACTORUM AG

**Beschreibung**

Gegenstand der Erfindung sind topische, kosmetische Zubereitungen zur Verbesserung des fettigen und unästhetischen Aussehens der Haare und der Haut, insbesondere zur Behandlung von stark fettendem Haar.

Die übermässig starke Absonderung der Talgdrüsen der Kopfhaut verleiht dem Haar ein fettiges Aussehen, das im allgemeinen als wenig ästhetisch angesehen wird. Es ist daher vielfach versucht worden, durch geeignete Mittel die Sekretion der Talgdrüsen zu normalisieren, um dem Haar wieder sein gesundes Aussehen zu geben. Zur Therapie der Seborrhoe wurden in der DE-OS 1 667 902 peroral verabreichbare Zubereitungen, welche Cysteamin-Derivate enthalten, vorgeschlagen. Es wurden zur Bekämpfung der Seborrhoe des behaarten Kopfes Shampoos mit Schwefel, Quecksilber oder Teerzusatz verwendet. Dabei hat sich gezeigt, dass diese bekannten Mittel bei längerer Anwendung häufig zu Nebenwirkungen führen, ohne dass wirklich befriedigende Ergebnisse im Hinblick auf Wirksamkeit oder anwendungstechnische Eigenschaften erzielt werden konnten. In der DE-OS 1 906 665 wurde schliesslich zur Behandlung der durch Seborrhoe verursachten Schuppenbildung N,N'-Diethyl-m-toluamid als Wirkstoff vorgeschlagen.

In der US-PS3 755 604 werden Phenylpentadiensäuren als Mittel zur Verhinderung der Sebumproduktion vorgeschlagen. Es hat sich jedoch gezeigt, dass weder N,N-Diethyl-m-toluamid noch Phenylpentadiensäure zufriedenstellende antiseborrhoische Wirkungen zeigt.

Es wurde nun gefunden, dass topische, kosmetische Zubereitungen mit einem Gehalt an Verbindungen der allgemeinen Formel:

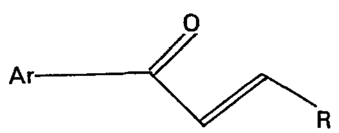

(I)

in der Ar für einen Phenylrest, der durch niedere Alkyl- oder Alkoxygruppen mit 1 - 4 Kohlenstoffatomen, Hydroxygruppen oder Halogene substituiert sein kann, einen Naphthylrest oder einen heteroaromatischen Rest wie Thienyl- oder Furyl- und R für Wasserstoff, eine Alkyl- oder Alkanoyl-Gruppe mit 1 - 4 Kohlenstoffatomen, eine Arylcarbonyl-Gruppe, wobei Aryl die gleiche Bedeutung wie Ar zukommt, oder einen durch niedere Alkyl- oder Alkoxygruppen mit 1 bis 4 Kohlenstoffatomen , Hydroxylgruppen oder Halogene substituierten Arylrest, stehen, eine ausgezeichnete Wirksamkeit bei der Behandlung seborrhoischer Haut und stark fettendem Haar aufweisen.

Die der Erfindung zugrunde liegenden Arylketonderivate sind literaturbekannt und zum Teil im Handel erhältlich. Für die Herstellung der ungesättigten Arylketone kann der Carbonylalkenyl-Rest in den Aromaten durch Friedel-Crafts-Synthese mit einem entsprechenden Säurehalogenid eingeführt werden. Eine weitere Möglichkeit der Herstellung der erfindungsgemässen Verbindungen ist die Aldolkondensation von Acetylaromaten entsprechenden Aldehyden. Die Doppelbindung kann auch durch Dehydrohalogenierung bzw. Dehalogenierung der entsprechenden gesättigten Halogenverbindungen eingeführt werden.

Im Sinne der Erfindung geeignete Verbindungen sind zum Beispiel:
1-Phenyl-prop-2-en-1-on, 1-Phenyl-but-2-en-1-on, 1-Phenyl-pent-2-en-1-on, 1-Phenyl-4,4-dimethyl-pent-2-en-1-on, ω-(p-Chlor-, p-Fluor-, p-Methoxy-, p-Methyl-benzal)-acetophenon, ω-(p-Chlor-benzal)-p-chlor-acetophenon, -2,4-dichlor-acetophenon, ω-Benzal-p-chlor-, -p-fluor-, -p-methoxy-, -p-hydroxy-, -2,4-dimethyl-acetophenon, cis-, trans-1,2-Dibenzoyl-ethen, trans-1,2-Di-(p-chlor-, -Di-(p-tert.butyl-, -Di-(p-methoxy-, -Di-(2,4-dimethyl-, -Di-(2,5-dimethyl-, -Di-(3,4-dimethyl-, -Di-(2,4-dihydroxy-benzoyl)-ethen.

Die erfindungsgemässen kosmetischen Mittel stellen Lösungen der einzusetzenden wirksamen Verbindungen der Formel (I) in Wasser, in Alkohol, in wässrig-alkoholischen Mischungen, in Öl, Suspensionen, Gelen, Emulsionen, Salben, Pasten oder Aerosolen dar. Die antiseborrhoischen Arylketonderivate können in fast alle zur Behandlung von Haut und Haaren üblichen kosmetischen Mittel eingearbeitet werden, wie zum Beispiel in Haarwässern, Haarshampoos, Haarkuren, Haarspülungen, oder auch in Hautlotionen und Schüttelmixturen. Neben den Verbindungen der Formel (I) enthalten die erfindungsgemässen Zubereitungen bekannte Träger und Hilfsstoffe wie Wasser, organische Lösungsmittel, oberflächenaktive Verbindungen, Öle und Fette, Wachse, Parfümöle, Farbstoffe, Konservierungsmittel und dergleichen. Eine vorteilhafte Anwendungsform zur Behandlung von stark fettendem Haar ist das Shampoo. Derartige Shampoos können neben dem sebosuppressiven Wirkstoff anionische, kationische, nichtionische oder amphotere Tenside, Farbstoffe, Duftstoffe, Verdickungsmittel oder Konditionierungsmittel enthalten.

Die erfindungsgemässen kosmetischen Zubereitungen enthalten die ungesättigten Arylketone in einer Menge von 0,01 - 20 Gew.-%, vzw. 0,1 - 1,0 Gew.-%, bezogen auf das Gewicht der gesamten Zubereitung. Die erfindungsgemässen Mittel können täglich angewendet werden; es werden jedoch bereits bei einmaliger wöchentlicher Anwendung zufriendestellende Ergebnisse erzielt. Die individuelle Dosis, die bei jeder Behandlung angewendet werden sollte, ist nicht kritisch. Nachteilige Nebeneffekte wurden nicht beobachtet. Das fettige Aussehen des Haares wird vermindert und das Nachfetten verzögert, wodurch die Möglichkeit einer normalen Haarpflege gegeben ist. Bei Einsatz der erfindungsgemässen Mittel in Form von Hautcremes oder Milchpräparaten oder Schüttelmixturen gelingt es, durch regelmässige Anwendung auf der Haut deren Aussehen dauerhaft zu verbessern.

Die nachfolgenden Beispiele sollen den Gegenstand der Erfindung näher erläutern.

*Beispiele*

Zunächst wird die Herstellung der ungesättigten

Arylketone anhand eines Herstellungsbeispiels erläutert.

*A) trans-1,2-Bis-(p-chlor-benzoyl)-ethen*

Zu der gerührten Suspension von 31 g (0,23 Mol) Aluminiumtrichlorid in 250 ml Chlorbenzol wurden bei Raumtemperatur 18 g (0,12 Mol) Fumarylchlorid langsam getropft; die Mischung wurde 1,3 Stunden bei Raumtemperatur und 1,3 Stunden bei 45°C weitergerührt. Nach Eintragen in Eis/Wasser, Ansäuern mit konz. HCl wurde mit 1,5 l Methylenchlorid ausgeschüttelt. Nach dem Verdampfen des Methylenchlorids wurde der Rückstand mit ca. 200 ml Ethanol gewaschen und aus Aceton umkristallisiert. Es wurden 27 g (75% d.Th.) trans-1,2-Bis-(p-chlor-benzoyl)-ethen vom Fp. 174 - 176°C erhalten.

Die folgenden Verbindungen wurden in analoger Weise erhalten:

*B) trans-1,2-Dibenzoyl-ethen*

Fp. 111°C

*C) trans-1,2-Bis-(2,4 dimethyl-benzoyl)-ethen*

Fp. 128 - 130°C

*D) trans-1,2-Bis-(2,5-dimethyl-benzoyl)-ethen*

Fp. 106 - 108°C

*E) trans-1,2-Bis-(3,4-dimethyl-benzoyl)-ethen*

Fp. 142 - 143°C

*F) trans-1,2-Bis-(p-methoxy-benzoyl)-ethen*

Fp. 162 - 164°C.

Die antiseborrhoische Wirkung der in den erfindungsgemässen kosmetischen Zubereitungen eingesetzten Verbindungen wurde durch nachfolgend beschriebene Tierversuche näher untersucht. Als Versuchtiere dienten männliche Wistar-Ratten von 220 - 230 g Körpergewicht. Beuteilt wurde visuell der Bräunungsgrad auf dem Rücken der dort geschorenen Ratten. Die Bräunung wird durch das braune Hautoberflächenlipid der Ratten hervorgerufen. Dieser Test geht von der Beobachtung aus, dass junge weibliche Ratten sowie männliche Ratten nach dem Waschen mit Tensidlösung bzw. einem Lipidlösungsmittel und auch männliche Ratten, die systemisch mit Östrogen behandelt wurden, nur die normale helle, rosafarbene Haut nach dem Scheren aufweisen; parallel dazu sind aus den abgeschnittenen Haaren nur noch vergleichsweise sehr geringe Lipidmengen zu extrahieren.

Zur Beurteilung der sebosuppressiven Wirksamkeit wurden die Prüfsubstanzen in Form einer 1%igen Lösung in Ethanol oder Ethanol/Aceton (1 : 1) jeweils 6 Ratten halbseitig auf das Rückenfell gepinselt. Die andere Seite wurde nur mit dem Lösungsmittel ohne Wirksubstanz behandelt (Kontrollseite).

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert.

Als 1. Kriterium wurde bewertet, ob die Mehrheit der Beurteiler richtig die behandelte Seite erkannt haben, wobei wie folgt differenziert wurde:

| Zeichen | Anteil der Beurteiler, die eine Wirkung erkannten |
|---|---|
| + + | 100% |
| + | >50%, 100% |
| 0 | ≤50% |

Als 2. Kriterium wurde der Unterschied zwischen rechter und linker Seite gewertet, wobei pro Beurteiler und Tier jeweils 1 Punkt zu vergeben war, und zwar in der Weise, dass die dunklere Seite mit 1 und die hellere mit 0 und bei Gleichheit beide Seiten mit 0,5 benotet wurden.

Als 3. Kriterium wurden ausserdem noch die Intensitätsunterschiede der Brauntöne nach folgender Skala bewertet:

| | |
|---|---|
| 3 Punkte | stark braun |
| 2 Punkte | mittel braun |
| 1 Punkt | schwach braun |
| 0 Punkte | keine Braunfärbung |

Signifikante Differenzen zwischen unbehandelter und behandelter Seite nach der zweiten Bewertungsmethode zeigen die lokale Wirksamkeit einer Substanz an. Nach der dritten Bewertungsmethode werden die Punktsummendifferenzen zwischen den unbehandelten Kontrolltieren und jeweils den behandelten und unbehandelten Seiten der Versuchstiergruppe gebildet, wobei wiederum signifikante Differenzen zwischen Kontrolltieren und der behandelten Seite der Versuchstiere die Wirkung einer Substanz deutlich machen.

Parallel dazu ist in der Regel auch ein deutlicher Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinfluss. Zur Differenzierung der Effekte gemäss Beurteilungsmethode 2 und 3 wurde das folgende Schema verwendet.

| Zeichen | Punktdifferenz |
|---|---|
| + + | sehr gross (>99,9% Wahrscheinlichkeit) |
| + | signifikant (≥95% Wahrscheinlichkeit) |
| (+) | deutlich aber (<95% Wahrscheinlichkeit) |

Die nachfolgende Tabelle zeigt die Bewertungsergebnisse nach dem vorgenannten Schema für die Verbindungen B und C.

*Bewertung der sebosuppressiven Effekte*

| Substanz | Bewertungsmethode 1 | 2 | 3 |
|---|---|---|---|
| B | ++ | ++ | ++ |
| C | + | (+) | (+) |
| 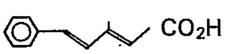 | 0 | 0 | + |
| US-PS 3 755 604 | | | |

Nachfolgend werden Beispiele für erfindungsgemässe topische Mittel zur Behandlung von stark fettendem Haar und seborrhoischer Haut angegeben.

*Hautcreme*

| | | |
|---|---|---|
| Selbstemulgierendes Gemisch aus Mono/Diglyceriden höherer gesättigter Fettsäuren mit Kaliumstearat® Dehydag | 16,0 | Gew.-T. |
| Cetylstearylalkohol mit ca. 12 Mol Ethylenoxid | 1,0 | » |
| 2-Octyldodecanol | 6,0 | » |
| Isopropylmyristat | 4,0 | » |
| Glycerin | 6,0 | » |
| Verbindung A | 0,7 | » |
| Wasser | 62,3 | » |

*Shampoo für fettendes Haar*

| | | |
|---|---|---|
| Ammoniumlaurylsulfat mit 33-35% Waschaktivsubstanz | 40,0 | Gew.-T. |
| Kokosfettsäurediethanolamid | 3,0 | » |
| Natriumchlorid | 2,0 | » |
| Natriumsulfat | 2,0 | » |
| Verbindung B | 0,6 | » |
| Parfümöl | 0,1 | » |
| Wasser | 52,3 | » |

**Patentansprüche**

1. Topische, kosmetische Zubereitungen, enthaltend eine Verbindung der allgemeinen Formel (I)

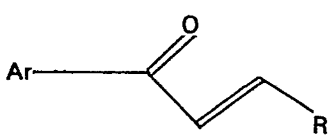

(I)

in der Ar für einen Phenylrest, der durch niedere Alkyl- oder Alkoxygruppen mit 1 - 4 Kohlenstoffatomen, Hydroxygruppen oder Halogene substituiert sein kann, einen Naphthylrest oder einen heteroaromatischen Rest wie Thienyl- oder Furyl- und R für Wasserstoff, eine Alkyl- oder Alkanoyl-Gruppe mit 1 - 4 Kohlenstoffatomen, eine Arylcarbonyl-Gruppe, wobei Aryl die gleiche Bedeutung wie Ar zukommt, oder einen durch niedere Alkyl- oder Alkoxygruppen mit 1 - 4 Kohlenstoffatomen, Hydroxylgruppen oder Halogene substituierten Arylrest, stehen, als antiseborrhoischen Wirkstoff neben üblichen Träger- und Hilfsstoffen.

2. Topische, kosmetische Zubereitungen nach Anspruch 1, dadurch gekennzeichnet, dass sie trans-1,2-Dibenzoylethen enthalten.

3. Topische, kosmetische Zubereitungen nach Anspruch 1 bis 2, dadurch gekennzeichnet, dass sie eine Verbindung der allgemeinen Formel (I) in einer Menge von 0,01 bis 20 Gew.-%, vzw. 0,1 - 1,0 Gew.%, bezogen auf die gesamte Zubereitung enthalten.

**Claims**

1. Topical cosmetic preparations containing a compound corresponding to the following general formula

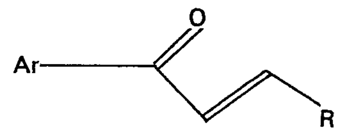

(I)

in which Ar is either a phenyl group which may be substituted by lower alkyl or alkoxy groups containing from 1 to 4 carbon atoms, hydroxy groups or halogens, or a naphthyl group or a heteroaromatic group, such as thienyl or furyl, and R represents hydrogen, an alkyl or alkanoyl group containing from 1 to 4 carbon atoms, an aryl carbonyl group, aryl having the same meaning as Ar, or an aryl group substituted by lower alkyl or alkoxy groups containing from 1 to 4 carbon atoms, hydroxyl groups or halogens, as antiseborrhoeic additive in addition to standard carriers and auxiliaries.

2. Topical cosmetic preparations as claimed in claim 1, characterized in that they contain trans-1,2-dibenzoyl ethene.

3. Topical cosmetic preparations as claimed in claim 1, characterized in that they contain a compound corresponding to general formula (I) in a quantity of from 0.01 to 20% by weight, preferably in a quantity of from 0.1 to 1.0% by weight, based on the preparations as a whole.

**Revendications**

1. Préparations cosmétiques topiques, contenant un composé de formule générale (I)

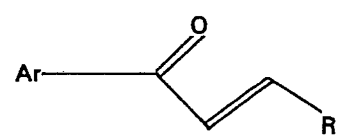

(I)

dans laquelle Ar représente ou un radical phényle qui peut être substitué par des groupes alcoyle ou alcoxy inférieurs ayant 1 à 4 atomes de carbone, par des groupes hydroxy ou des halogènes, ou un radical naphtyle ou un radical hétéroaromatique comme le radical thiényle ou furyle, et R représente

de l'hydrogène, un groupe alcoyle ou alcanoyle ayant 1 à 4 atomes de carbone, un groupe arylcarbonyle, l'aryle ayant la signification qui revient à Ar, ou un radical aryle substitué par des groupes alcoyle ou alcoxy inférieurs ayant 1 à 4 atomes de carbone, par des groupes hydroxyle ou des halogènes, en tant que substance active antiséborrhéique à côté des matières de support et auxiliaires usuelles.

2. Préparations cosmétiques topiques, suivant la revendication 1, caractérisées en ce qu'elles contiennent du trans-1,2-dibenzoyl-éthène.

3. Préparations cosmétiques topiques, suivant les revendications 1 ou 2, caractérisées en ce qu'elles contiennent un composé de formule générale (I) en une quantité de 0,01 à 20% en poids, de préférence 0,1 à 1,0% en poids par rapport à l'ensemble de la préparation.